# EUROPÄISCHE PATENTANMELDUNG

(11) **EP 1 208 792 A1**
(43) Veröffentlichungstag der Anmeldung: **29.05.2002**
(21) Anmeldenummer: 00811094.2
(22) Anmeldetag: 17.11.2000
(51) Int. Cl.: A61B 3/16, A61B 3/125

(54) **Vorrichtung und Verfahren zur Untersuchung und/oder Behandlung eines Auges**

(71) Anmelder: HAAG-STREIT AG, CH-3098 Köniz (CH)
(72) Erfinder: Fischer, Konrad, 3007 Bern (CH); Teijido, Juan Manuel, 2012 Auvernier (CH)
(74) Vertreter: Roshardt, Werner Alfred, Dipl.-Phys.

(57) **Zusammenfassung**

Eine Vorrichtung (1.3) zur Untersuchung und/oder Behandlung eines Auges (15) weist einen Basiskörper (2.3) und einen Ansetzkörper (3.3) auf, welche zusammengesetzt und auf das Auge aufgesetzt werden können, wobei zwecks Indexmatching eine gelartige Flüssigkeit (14) zwischen die beiden Körper eingebracht wird. Damit die Luft zwischen den beiden Körpern oder auch überschüssige Flüssigkeit entweichen kann, ist die Innenseite des Ansetzkörpers mit einer Mehrzahl von Erhebungen (13.1) versehen, welche einen minimalen Abstand zwischen den beiden Körpern gewährleisten. Da bei einer Untersuchung/Behandlung nur der Ansetzkörper, nicht aber der Basiskörper mit dem Auge bzw. dem Patienten in Kontakt kommt, muss auch nur der Ansetzkörper sterilisierbar ausgebildet sein, wobei eine einmalige, in den Produktionsprozess integrierte Sterilisation ausreicht. Als Wegwerfartikel konzipiert kann er aus preiswerten Materialen auf einfache Art und Weise gefertigt werden. Der Basiskörper muss hingegen nur desinfizierbar sein. Diese Aufteilung der Vorrichtung erlaubt es des Weiteren, eine Mehrzahl von unterschiedlich ausgebildeten Basiskörpern mit einem einzigen Ansetzkörper bzw. eine Mehrzahl von unterschiedlich ausgebildeten Ansetzkörpern mit einem einzigen Basiskörper zu verwenden. Mit nur wenigen verschiednen Basis- bzw. Ansetzkörpern kann somit eine Vielzahl verschiedener Augenuntersuchungen bzw. -behandlungen durchgeführt werden.

## Beschreibung

### Technisches Gebiet

Die Erfindung betrifft eine Vorrichtung zur Untersuchung und/oder Behandlung eines Auges, wobei sie auf das Auge aufzusetzen ist.

### Stand der Technik

Aus dem Stand der Technik sind verschiedene Vorrichtungen zur Augenuntersuchung bzw. -behandlung bekannt, welche zur Untersuchung bzw. Behandlung des Auges direkt auf dieses aufgesetzt werden. Der Einfachheit halber ist im Folgenden unter dem Begriff Untersuchung jeweils auch eine entsprechende Behandlung des Auges zu verstehen.

Zu solchen Vorrichtungen zur Augenuntersuchung gehören beispielsweise sogenannte Kontaktgläser, welche es erlauben, durch die Pupille des Auges hindurch den Augenhintergrund oder andere Bereiche im Augeninnern zu untersuchen oder zu behandeln. Hierzu gehören aber auch andere Vorrichtungen, wie beispielsweise Tonometer, welche zur Augendruckmessung direkt mit dem Auge in Kontakt gebracht werden.

Derartige Vorrichtungen werden in vielen verschiedenen Varianten hergestellt und verkauft. So gibt es beispielsweise verschiedene Grössen von Kontaktgläsern für Erwachsene oder Kinder. Je nach gewünschter Anwendung können sie auch einen oder mehrere, seitlich angeordnete Spiegel aufweisen, mit welchem sich periphere Bereiche des Augeninnern untersuchen lassen, welche ohne Spiegel nicht sichtbar wären. Weiter gibt es auch Kontaktgläser, welche im jenem Bereich, welcher mit dem Auge in Kontakt gebracht wird, einen sogenannten Skleraring aufweisen, welcher in Untersuchungsposition derart auf dem Auge aufliegt, dass er von den Augenlidern bedeckt bzw. durch diese auf dem Auge gehalten wird.

Die bekannten Vorrichtungen weisen den Nachteil auf, dass zum Einen für verschiedene Untersuchungen unterschiedliche Ausführungsformen der Vorrichtung bereit gehalten werden müssen. Und um zu verhindern, dass das Auge bei der Untersuchung verschmutzt und damit auch die Gefahr von Infekten erhöht wird, müssen die Vorrichtungen zum Andern vor jedem Gebrauch desinfiziert bzw. sterilisiert werden. Dies hat allerdings Abnützungserscheinungen zur Folge, weshalb die Vorrichtungen von Zeit zu Zeit ersetzt werden müssen.

### Darstellung der Erfindung

Aufgabe der Erfindung ist es, eine Vorrichtung der genannten Art anzugeben, welche die Nachteile der aus dem Stand der Technik bekannten Vorrichtungen vermeidet und auf einfache Art und Weise eine Übertragung von Infekten oder Reizelementen verhindert. Auch soll insbesondere eine kostengünstige und einfache Möglichkeit zur Augenuntersuchung geboten werden.

Die Lösung der Aufgabe ist durch die Merkmale des Anspruchs 1 definiert. Gemäss der Erfindung weist die zur Untersuchung und/oder Behandlung eines Auges auf das Auge aufzusetzende Vorrichtung einen Basiskörper und einen mit dem Basiskörper zusammensetzbaren Ansetzkörper auf, wobei die Vorrichtung zur Untersuchung und/oder Behandlung des Auges mit dem Ansetzkörper auf das Auge aufzusetzen ist.

Diese Aufteilung der Vorrichtung in einen Basiskörper und einen Ansetzkörper hat viele Vorteile. So kann der Basiskörper fakultativ beispielsweise mit besonderen optischen Elementen zur Augenuntersuchung wie einem oder mehreren Spiegeln oder bestimmten Linsenanordnungen versehen werden. Im Basiskörper steckt somit ein Grossteil des mechanischen bzw. optischen Aufwandes. Der Ansetzkörper benötigt deshalb keine optisch wirksamen Elemente, sondern besteht typischerweise nur aus einer dünnen Schicht von optisch transparentem Material. Er ist auf einfache und kostengünstige Art in grossen Mengen fertigbar. Er kann einerseits als Wegwerfartikel für eine einmalige oder für einige wenige Verwendungen ausgebildet sein. Andererseits kann er für eine mehrfache Verwendung ausgebildet sein, wobei er auch hier kostengünstig durch einen neuen ersetzbar ist, wenn beispielsweise der Verschleiss infolge von Desinfektion oder Sterilisation zu gross geworden ist. Allerdings ist darauf zu achten, dass der Ansetzkörper in jenem Bereich, der mit dem Auge in Kontakt kommt, derart ausgebildet ist, dass das Auge nicht verletzt wird. So sollte beispielsweise jene Fläche des Ansetzkörpers, mit welcher er auf das Auge aufgesetzt wird, keine Rillen oder Kanten aufweisen, sondern eher glatt ausgebildet und an die Augenoberfläche angepasst sein.

Weiter können beispielsweise unterschiedliche Ansetzkörper an ein- und denselben Basiskörper angesetzt werden. Hierdurch sind auf kostengünstige Art verschiedene Untersuchungen möglich, wofür beim Stand der Technik verschiedene, jeweils teure Vorrichtungen notwendig sind.

Selbstverständlich ist es auch umgekehrt möglich, einen einzigen Ansetzkörper bzw. eine einzige Art von Ansetzkörpern zusammen mit verschiedenen Basiskörpern zu verwenden, wodurch sich weitere Vorrichtungs-Varianten abdecken lassen.

Durch eine Kombination von einigen wenigen Varianten von Ansetzkörpern mit einigen wenigen Varianten von Basiskörpern lassen sich praktisch sämtliche Vorrichtungs-Varianten zusammensetzen, was eine vielfältige und dennoch einfache und kostengünstige Augenuntersuchung ermöglicht.

Die äussere Form des Basiskörpers kann im Prinzip beliebig sein. Typischerweise weist er jedoch auf einer Seite eine plane Oberfläche auf, durch welche hindurch das Auge untersucht werden kann. Weiter weist der Basiskörper vorzugsweise eine Kontaktfläche auf, welche sich typischerweise gegenüber der planen Oberfläche befindet. Diese Kontaktfläche kann sowohl plan, als auch beliebig konvex oder konkav, bzw. innerhalb bestimmter Grenzen beliebig geformt sein.

Der Ansetzkörper ist bevorzugt mit einer weiteren Kontaktfläche versehen, welche im Wesentlichen formgleich zur Kontaktfläche des Basiskörpers ausgebildet ist, wobei die beiden Kontaktflächen bei der zusammengesetzten Vorrichtung im Wesentlichen parallel zueinander liegen. Im Wesentlichen formgleich heisst, dass die Kontaktflächen nicht identisch, sondern derart ausgebildet sind, dass sie ungefähr die gleiche Form und etwa die selbe Grösse aufweisen, damit sie miteinander in Kontakt gebracht werden können. Die beiden Kontakflächen sind beispielsweise plan in der Form eines Polygons mit ungefähr den gleichen Seitenverhältnissen und Flächen ausgebildet oder sie sind beide rund und weisen etwa den gleichen Durchmesser auf. Weist die Kontaktfläche des Basiskörpers hingegen eine Wölbung nach innen oder nach aussen auf, ist die Kontaktfläche des Ansetzkörpers mit einer entsprechenden Wölbung nach aussen bzw. innen versehen.

Im Wesentlichen parallel heisst, dass plane Kontaktflächen in parallelen Ebenen zu liegen kommen und dass bei gewölbten Kontakflächen die Wölbung des Ansetzkörpers in etwa der inversen Wölbung des Basiskörpers entspricht, damit sie quasi ineinander gesetzt werden können. Allerdings können die Krümmungsradien der Wölbungen durchaus leicht unterschiedlich sein, damit sich die Kontakflächen nur an einzelnen Stellen berühren. Auf diese Weise entstehen zwischen den Kontaktflächen unter Umständen mit Luft gefüllte Zwischenräume.

Bei der Untersuchung des Auges sind derartige Zwischenräume jedoch störend, da Lichtstrahlen an solchen Grenzübergängen von einem Festkörper zu Luft bzw. umgekehrt starken optischen Störungen wie Beugung, Reflexion und Streuung unterworfen sind. Um solche Störungen zu vermeiden bzw. zu verringern, wird nun beim Zusammensetzen der Vorrichtung vorzugsweise ein optisch transparentes, verformbares Zwischenmaterial zwischen den Basiskörper und den Ansetzkörper eingebracht. Dieses Material ist beispielsweise gelartig oder fliessfähig ausgebildet. Es können auch lediglich nur elastisch oder plastisch verformbare Materialien verwendet werden. Das Material soll lediglich derart verformbar sein, dass es sich beim Zusammensetzen gut an die Kontaktflächen anschmiegt, wobei die Kontaktflächen derart geformt sind, dass beim Zusammensetzen keine bzw. nur wenige oder kleine Luftblasen eingeschlossen werden. Für das Zwischenmaterial wird ein Werkstoff verwendet, dessen Brechungsindex in etwa mit den Brechungsindizes von Basiskörper und Ansetzkörper übereinstimmt. Je genauer die Brechungsindizes von Basiskörper, Ansetzkörper und verformbarem Material übereinstimmen, desto geringer sind die resultierenden Störungen. Dadurch können die Kontaktflächen mit einer niedrigen optischen Güte und damit kostengünstig gefertigt werden. Teure Schleif- und/oder andere Vergütungsvorgänge wie beispielsweise eine Antireflexvergütung, wie sie bei der Herstellung optischer Oberflächen mit hoher Qualität notwendig sind, entfallen.

Das Einbringen dieses verformbaren Materials zwischen Basiskörper und Ansetzkörper hat noch einen weiteren Vorteil: Durch die adhäsionsbedingten Haftkräfte zwischen dem verformbaren Material und dem Basiskörper einerseits bzw. dem verformbaren Material und dem Ansetzkörper andererseits, bleiben der Basiskörper und der Ansetzkörper ohne Einwirkung anderer, äusserer Kräfte aneinander haften. D. h. je nach Ausbildung der beiden Körper, beispielsweise bei einem Ansetzkörper mit geringem Gewicht, ist es nicht notwendig, zwischen Basis- und Ansetzkörper zusätzliche Verbindungsmittel vorzusehen. Für die Augenuntersuchung durch einen Benutzer, welcher zur Positionierung der zusammengesetzten Vorrichtung auf dem Auge diese am Basiskörper ergreift, reichen die durch die Adhäsion des verformbaren Materials mit den Kontaktflächen von Basis- bzw. Ansetzkörper entstehenden Haftkräfte aus, den Ansetzkörper am Basiskörper zu halten.

Um beispielsweise den Kammerwinkel oder andere periphere Bereiche des Augenhintergrundes mit bekannten Vorrichtungen zu untersuchen, wird beispielsweise ein Kontakglas verwendet, welches nicht symmetrisch aufgebaut ist, sondern auf einer Seite einen Spiegel aufweist. Schaut man von oben in ein solches, auf ein Auge aufgesetzes Kontakglas, kann via den seitlich im richtigen Winkel angebrachten Spiegel der Kammerwinkel untersucht werden. Um die peripheren Bereiche des Auges vollständig untersuchen zu können, muss das Kontaktglas gedreht werden, wobei es nicht vom Auge des Patienten entfernt wird. Bei bekannten Vorrichtungen aus dem Stand der Technik, besteht hierbei eine erhöhte Verletzungsgefahr. Es kann beispielsweise die Hornhaut des Auges beschädigt, oder eine zwischen Hornhaut und Kontaktglas eingeklemmte Wimper ausgerissen werden. Eine derartige Drehung ist darüberhinaus für den Patienten äusserst unangenehm.

Bei einer bevorzugten Ausführungsform der erfindungsgemässen Vorrichtung sind der Basiskörper und der Ansetzkörper derart ausgebildet, dass sie im zusammengesetzten Zustand gegeneinander verdrehbar sind. D. h. sollen periphere Bereiche eines Auges vollständig untersucht werden, kann, analog zum Kontaktglas aus dem Stand der Technik, ein Basiskörper mit einem entsprechenden Spiegel verwendet werden. Dieser wird mit einem passenden Ansetzkörper zur Vorrichtung zusammengesetzt und die Vorrichtung wird mit dem Ansetzkörper auf das zu untersuchende Auge aufgesetzt. Indem nun der Basiskörper gegen den Ansetzkörper, inbesondere um die optische Achse der beiden Körper, verdreht wird, können die peripheren Bereiche vollständig untersucht werden, ohne dass hierbei der Ansetzkörper mitgedreht wird. Der Ansetzkörper, der als einziges Teil der Vorrichtung Kontakt mit dem Auge hat, wird hierbei nicht bewegt.

Bei einer möglichen Ausbildungsform der Vorrichtung, ist sowohl der Basiskörper als auch der Ansetzkörper rotationssymmetrisch ausgebildet. Der Basiskörper weist beispielsweise die Form eines Kegels mit abgeschnittener Spitze und der Ansetzkörper die Form eines Trichters mit abgeflachtem Boden auf, wobei sich die Kontaktfläche des Basiskörpers aussen im Bereich der abgeflachten Spitze und die Kontaktfläche des Ansetzkörpers auf der Innenseite des abgeflachten Trichterbodens befinden. Die Abmessungen von Basiskörper und Ansetzkörper sind dabei derart gewählt, dass der Basiskörper in den Trichter eingesetzt und auf diese Weise der Kontakt zwischen den Kontaktflächen hergestellt werden kann.

Damit zwischen dem Basiskörper und dem Ansetzkörper ausserhalb der Kontaktflächen, d. h. beispielsweise im Bereich der Aussenwände des Basiskörpers bzw. der Seitenwände des Trichters, ein gewisser Mindestabstand gewahrt bleibt, weist entweder der Basiskörper selber oder der Ansetzkörper ausserhalb der jeweiligen Kontaktfläche Distanzierungselemente, beispielsweise eine Mehrzahl von Erhebungen auf. Diese können z.B. in Form einer Mehrzahl von Noppen, in Form von länglich verlaufenden Stegen in der Art eines Kammes oder in Form von flächenmässigen Erhebungen ausgebildet sein.

Eine weitere, bevorzugte Ausführungsform der Vorrichtung ist derart ausgebildet, dass, wenn sie zusammengesetzt und auf das zu untersuchende Auge eines Patienten aufgesetzt ist, der Patient nur mit dem Ansetzkörper in Kontakt kommt. D. h. das Auge selber wie auch die Augenlider oder die Wimpern des Patienten kommen, eine entsprechende Handhabung vorausgesetzt, nicht mit dem Basiskörper in Kontakt.

Der Basiskörper muss, da er nicht in direkten Kontakt mit dem Auge bzw. dem Patienten kommt, nach Gebrauch lediglich desinfiziert, nicht jedoch sterilisiert werden. Aus diesem Grund kann er aus Materialen wie beispielsweise Kunststoff gefertigt werden, welche nicht nur kostengünstiger sind, sondern sich auch entsprechend einfacher wie beispielsweise Glas verarbeiten lassen.

Bei der oben erwähnten trichterförmigen Ausbildung des Ansetzkörpers ist beispielsweise der Trichterrand so breit, dass eine Berührung des Basiskörpers mit dem Auge bzw. einem Augenlid oder den Wimpern ausgeschlossen werden kann.

Wie bereits erwähnt, können Basis- und Ansetzkörper derart ausgebildet sein, dass sie nach dem Zusammensetzen aneinander haften, da beispielsweise ihre Oberflächen derart ausgebildet sind oder sich zwischen den Oberflächen ein verformbares Material befindet, dass die Adhäsionskräfte ausreichen, um sie zusammenzuhalten. Bei einer weiteren, bevorzugten Ausführungsform der erfindungsgemässen Vorrichtung zur Augenuntersuchung sind Basis- und der Ansetzkörper derart ausgebildet, das sie durch eine lösbare, form- und/oder kraftschlüssige Verbindung zusammengehalten werden. Denkbar ist beispielsweise eine Schraubverbindung, eine Art Kniehebelverschluss, ein Rastmechanismus zur Herstellung einer Klickverbindung oder eine beliebige andere Verbindungsart.

Durch die Verwendung einer lösbaren Verbindung zwischen Basis- und Ansetzkörper ist gewährleistet, dass der Ansetzkörper nach Gebrauch, d. h. nach der Untersuchung eines Auges vom Basiskörper abgenommen werden kann. Danach kann er beispielsweise weggeworfen werden oder er ist aus einem Material gefertigt, das eine Sterilisierung des Ansetzkörpers, beispielsweise mit einem Autoklaven, erlaubt.

Der Basiskörper ist, wie bereits erwähnt, derart ausgebildet, dass er eine ophtalmologische, insbesondere eine optische Untersuchung des Auges erlaubt. Er ist beispielsweise aus einem optisch transparenten Material gefertigt, durch welches Lichtstrahlen sowohl in das Auge hinein, als auch aus dem Auge hinaus gelangen können. Um Fremdlicht fernzuhalten bzw. um störende Reflexionen an den seitlichen Begrenzungsflächen zu vermindern, können diese beispielsweise dunkel eingefärbt oder mit einem entsprechenden Material ummantelt werden.

Zur Untersuchung der seitlichen Bereiche des Augenhintergrundes bzw. des Kammerwinkels weist der Basiskörper bevorzugt einen oder eine Mehrzahl von Spiegeln auf. Die Spiegel sind hierbei derart platziert, dass die Sicht auf den zentralen Augenhintergrund offen bleibt. Jeder Spiegel weist weiter einen bestimmten Neigungswinkel auf, welcher die Untersuchung eines ganz bestimmten Bereiches des Augeninnern erlaubt. Bei mehreren Spiegeln weisen meist alle Spiegel einen unterschiedlichen Neigungswinkel auf.

Die beschriebenen Basis- und Ansetzkörper können natürlich auch derart ausgebildet sein, dass sie nicht nur zur Untersuchung, sondern auch zur Behandlung eines Auges, beispielsweise mit einem Laserstrahl, eingesetzt werden können.

Wie ebenfalls bereits erwähnt, wird die Vorrichtung zur Untersuchung des Auges jeweils mit dem Ansetzkörper auf das Auge aufgesetzt. Der Ansetzkörper weist daher bevorzugt eine Berührungsfläche auf, welche im Wesentlichen an einen Oberflächenbereich des zu untersuchenden Auges angepasst ist. D. h. die Berührungsfläche weist eine Wölbung auf, welche in etwa der Wölbung der Hornhaut des zu untersuchenden Auges entspricht. Die Berührungsfläche liegt zumeist auf der Kontaktfläche gegenüberliegenden Seite des Ansetzkörpers.

Zur Untersuchung von unterschiedlich grossen Augen mit demzufolge unterschiedlichen Wölbungen, beispielsweise Kinderaugen im Gegensatz zu Augen Erwachsener, können mit demselben Basiskörper verschieden gewölbte Aufsetzkörper verwendet werden.

Die Form der Berührungsfläche kann praktisch frei gewählt werden, wobei eine runde Form bevorzugt wird. Auch die Grösse der Berührungsfläche kann, in gewissen Grenzen, frei gewählt werden. Die untere Grenze für die Grösse ist einerseits durch die Lichtmenge bestimmt, die zur Untersuchung notwendig ist und anderseits sollte sie auch nicht so klein gewählt werden, dass eine Verletzungsgefahr für das Auge besteht. Bei runden Berührungsflächen sollte der Durchmesser beispielsweise auch nicht grösser gewählt werden als der grösste Durchmesser des sichtbaren Teils des Auges bei geöffneten Augenlidern. Die Berührungsfläche typischer Ansetzkörper entspricht in etwa der Grösse der Iris.

Es gibt jedoch Personen, welche beispielsweise eher unruhig sind oder bei welchen der Abstand der Augenlider bei geöffnetem Auge kleiner ist als bei anderen. Bei solchen Personen ist es schwierig, die auf das Auge aufgesetzte Vorrichtung in korrekter Position zu halten, ohne beispielsweise die Augenlider mit entsprechenden Klammern zu fixieren. Um diese für den Patienenten unangenehme Prozedur zu vermeiden, werden Ansetzkörper verwendet, deren Berührungsfläche bevorzugt derart ausgebildet ist, dass sie Randbereiche aufweist, welche beim Aufsetzen auf das Auge zumindest teilweise unter zumindest ein Augenlid des Patienten schiebbar sind und der Ansetzkörper auf diese Weise von dem oder den Augenlidern in korrekter Position gehalten wird. Die Berührungsfläche weist beispielsweise rund um den zur Untersuchung transparenten Bereich eine Art Ring, einen sogenannten Skleraring auf, welcher beim Aufsetzen des Ansetzkörpers auf das Auge unter die Augenlider geschoben wird.

Zur Gewährleistung einer korrekten und möglichst einfachen Handhabung der Vorrichtung werden die Ansetzkörper nach der Fertigung vorzugsweise gebrauchsfertig in einem Behälter zur Lagerung oder für den Transport untergebracht. Die Ansetzkörper werden zuvor typischerweise sterilisiert und einzeln verpackt. Hierfür eignen sich beispielsweise sogenannte Blista-Packungen, bei welchen sich die einzelnen Gegenstände in jeweils einer eigenen Kammer eines Behälters befinden, welche mit jeweils einer Folie abgedeckt sind.

Der Behälter ist beispielsweise derart ausgebildet, dass er das Aufstecken des Ansetzkörpers auf den Basiskörper ohne eine Berührung des Ansetzkörpers durch den Benutzer ermöglicht. Der Benutzer zieht zunächst die Folie von der Kammer mit dem gewünschten Ansetzkörper ab, wobei der sterile Ansetzkörper in einer speziellen Halterung liegt. Diese ermöglicht es dem Benutzer, das verformbare Material, beispielsweise eine gelartige Flüssigkeit auf die Kontaktfläche aufzubringen und anschliessend den zu verwendenden Basiskörper auf den Ansetzkörper aufzustecken bzw. in diesen hineinzusetzen und die Vorrichtung auf diese Weise zusammenzusetzen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Ein zusammengesetztes, erfindungsgemässes Kontaktglas zur Augenuntersuchung in schematischer, perspektivischer Darstellung;
- Fig. 2: eine Schnittansicht des Kontaktglases aus Fig. 1;
- Fig. 3: eine nicht zusammengesetzte, andere Variante des erfindungsgemässen Kontaktglases;
- Fig. 4: eine zusammengesetzte, weitere Variante des Kontaktglases mit einer Klickverbindung zwischen Basis- und Ansetzkörper;
- Fig. 5: ein Kontaktglas mit aufgebrachtem, gelartigen Material zur Brechungsindex-Anpassung vor dem Zusammensetzen;
- Fig. 6: das Kontaktglas aus Fig. 5 nach dem Zusammensetzen;
- Fig. 7: eine Schnittansicht des Kontaktglases aus Fig. 6;
- Fig. 8: das auf ein Auge aufgesetzte Kontaktglas aus Fig. 6;
- Fig. 9: ein auf ein Auge aufgesetztes Kontaktglas mit einem einen Skleraring aufweisenden Ansetzkörper;
- Fig. 10: ein vergrössert dargestellter Ausschnitt des Kontaktglases aus Figur 8;
- Fig. 11: ein auf ein Auge aufgesetztes Kontaktglas mit einem einen Spiegel aufweisenden Basiskörper und
- Fig. 12: das Kontaktglas aus Figur 11 von oben gesehen.

Grundsätzlich sind in den Figuren gleiche Teile mit gleichen Bezugszeichen versehen.

### Wege zur Ausführung der Erfindung

Anhand der Figuren sollen einige bevorzugte Varianten der Erfindung näher erläutert werden.

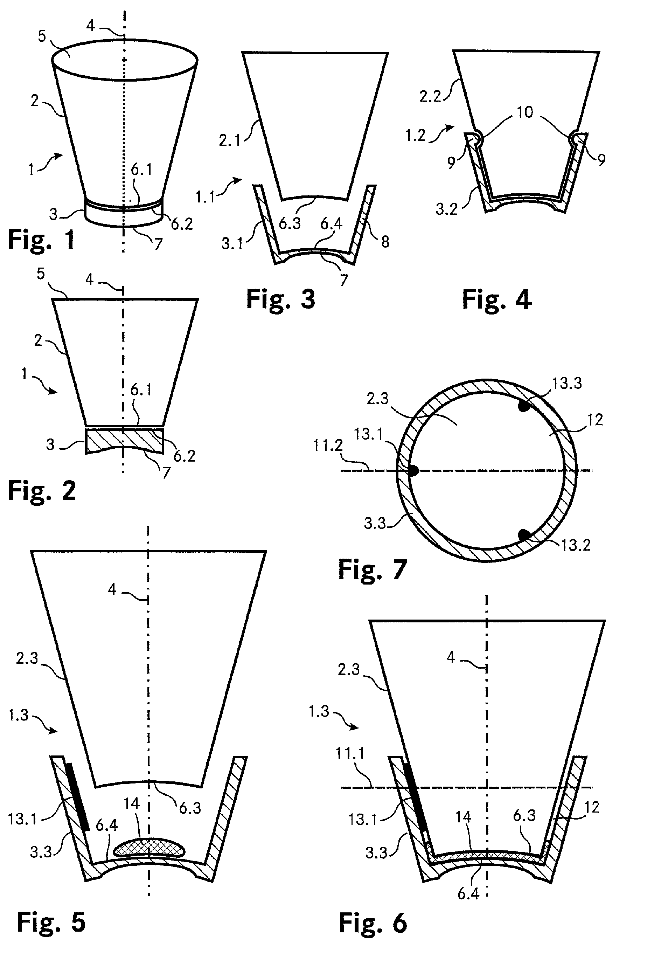

Figur 1 zeigt eine perspektivische, schematische Darstellung eines erfindungsgemässen Kontaktglases 1 zur Untersuchung des Inneren eines Auges. Es besteht aus einem Basiskörper 2 und einem Ansetzkörper 3, welche zusammengesetzt dargestellt sind. Das Kontaktglas 1 ist rotationssymmetrisch zur Rotationsachse 4 ausgebildet. In Figur 2 ist ein Längsschnitt durch das Kontaktglas 1 dargestellt, wobei die Schnittebene die Rotationsachse 4 enthält und das Kontaktglas 1 in zwei identische Hälften teilt.

Der mit dem Ansetzkörper 3 zusammengesetzte Basiskörper 2 bildet in diesem Fall ein Kontaktglas 1. Ebensogut könnten sie zusammengesetzt auch einen Tonometermesskörper bilden, der unter einem bestimmten Druck auf das Auge aufgesetzt wird. Aus der messbaren Verformung der Augenoberfläche und dem Druck lässt sich anschliessend der Augeninnendruck bestimmen. Die nachfolgende Beschreibung der Figuren ist, wo dies Sinn macht, entsprechend auch als Beschreibung von Vorrichtungen zur Messung des Augeninnendruckes zu verstehen, bei welchen der Basiskörper als Tonometermesskörper zu interpretieren ist.

Der Basiskörper 2 weist auf seiner Oberseite eine plane, in diesem Fall kreisrunde, vorzugsweise senkrecht zur Rotationsachse 4 verlaufende Oberfläche 5 auf. Der planen Oberfläche 5 gegenüber befindet sich die ebenfalls kreisrunde Kontaktfläche 6.1 des Basiskörpers 2. Der Ansetzkörper 3 weist auf seiner Oberseite ebenfalls eine wiederum kreisrunde Kontaktfläche 6.2 und auf seiner Unterseite eine ebenso kreisrunde Berührungsfläche 7 auf. Sowohl der Basiskörper 2, als auch der Ansetzkörper 3 sind aus optisch transparentem Material wie beispielsweise Glas oder aus entsprechenden Kunststoffen wie beispielsweise Plexiglas gefertigt, so dass sie für Licht transparent sind und eine Betrachtung durch die Körper hindurch erlauben.

Damit das Kontaktglas auch ohne ein zwischen die Kontaktflächen 6.1, 6.2 einzubringendes Mittel für ein Indexmatching, d.h. eine Anpassung der Brechungsindizes der verschiedenen Materialen, verwendet werden kann, sind die Kontaktflächen 6.1, 6.2 mit einer hohen optischen Qualität geschliffen, poliert und/oder mit einer Antireflexbeschichtung vergütet. Damit könnten nicht nur störende Reflexionen an den Kontaktflächen 6.1, 6.2 vermindert, sondern auch eine gewisse, durch Adhäsion zwischen den Kontaktflächen 6.1, 6.2 hervorgerufene Haftung des Ansetzkörpers 3 am Basiskörper 2 bzw. umgekehrt erreicht werden.

Zur Untersuchung eines Auges wird das Kontaktglas 1 von einem Benutzer mit der Berührungsfläche 7 im Bereich der Pupille des zu untersuchenden Auges auf dieses aufgesetzt. Danach kann der Benutzer, indem er mehr oder weniger senkrecht auf die plane Oberfläche 5 bzw. durch das Kontaktglas 1 hindurch blickt, das Innere des zu untersuchenden Auges beobachten.

Selbstverständlich kann das Kontaktglas 1 auch zur Behandlung des Auges, beispielsweise für Laseranwendungen, verwendet werden.

Figur 3 zeigt eine Schnittansicht durch eine andere Variante eines Kontaktglases, wobei die Kontaktfläche 6.3 des Basiskörpers 2.1 konvex, d. h. nach innen gewölbt, ausgebildet ist. Entsprechend ist die Kontaktfläche 6.4 des Ansetzkörpers 3.1 konkav, d. h. nach aussen gewölbt, ausgebildet. Die Wölbungen der beiden Kontaktflächen 6.3, 6.4 müssen nicht exakt übereinstimmen, entsprechen sich jedoch ungefähr.

Der Ansetzkörper 3.1 ist trichterförmig ausgebildet. Er weist eine rundum laufende Seitenwand 8 auf, welche den Basiskörper bis etwa hinauf zur Hälfte bedeckt. Damit wird erreicht, dass der Basiskörper 2.1, wenn das Kontaktglas 1.1 auf ein Auge aufgesetzt wird, weder das Auge selber, noch ein Augenlid, die Wimpern oder sonst irgendwo den Patienten berührt.

In Figur 4 ist eine mögliche Variante dargestellt, wie der Basiskörper 2.2 mit dem Ansetzkörper 3.2 lösbar verbunden werden kann. Der wiederum trichterförmig ausgebildete Ansetzkörper 3.2 weist am oberen Rand des Trichters auf der Innenseite eine vollständig oder teilweise umlaufende kammartige Erhebung 9 auf, welche beim Zusammensetzen mit dem Basiskörper 2.2 in eine entsprechende Ringnut 10 am Basiskörper 2.2 einrastet. Die Ringnut 10 kann entweder ebenfalls vollständig um den Basiskörper 2.2 laufen oder sie kann auch unterbrochen sein, um beispielsweise eine gegenseitige Verdrehung von Basiskörper und Ansetzkörper zu verhindern.

Die Figuren 5, 6 und 7 zeigen ein weiteres Kontaktglas 1.3. Figur 5 eine Schnittansicht des nicht zusammengesetzten Kontaktglases 1.3, Figur 6 eine Schnittanschicht des zusammengesetzten Kontaktglases 1.3 und Figur 7 eine weitere Schnittansicht des zusammengesetzten Kontaktglases 1.3 entlang einer senkrecht zur Rotationsachse 4 liegenden Schnittebene 11.1.

Um zwischen dem Basiskörper 2.3 und dem wiederum trichterförmig ausgebildeten Ansetzkörper 3.3 des zusammengesetzten Kontaktglases 1.3 einen Zwischenraum 12 zu gewährleisten, sind auf der Innenseite des Ansetzkörpers 3.3 eine Mehrzahl von länglichen, von oben nach unten verlaufenden Stegen 13.1, 13.2, 13.3. vorgesehen. Da es sich bei den Figuren 5 und 6 um Längsschnitte entlang der Schnittebene 11.2 handelt, ist jeweils nur ein Steg 13.1 sichtbar. Deren Abmessungen sind derart gewählt, dass beim zusammengesetzten Kontaktglas 1.3 ein bestimmter Abstand zwischen den beiden Kontaktflächen 6.3, 6.4 des Basiskörpers 2.3 bzw. des Ansetzkörpers 3.3 erreicht wird.

Der Zwischenraum 12 könnte natürlich auch erreicht werden, indem derartige Stege oder andere Erhebungen auf der Aussenseite des Basiskörpers 2.3 aufgebracht würden. Oder die Innenseite des Ansetzkörpers 3.3 bzw. die Aussenseite des Basiskörpers 2.3 würden mit entsprechenden Nuten versehen. Diese hätten jedoch den Nachteil, dass sie schneller verschmutzen würden.

Die Figuren 5 und 6 zeigen noch einen weiteren Aspekt. Vor dem Zusammensetzen von Basiskörper 2.3 und Ansetzkörper 3.3 zum Kontaktglas 1.3 kann zunächst eine bestimmte Menge einer optisch transparenten, verformbaren, beispielsweise gelartigen Flüssigkeit 14 auf die Kontaktfläche 6.4 aufgebracht werden. Beim Zusammensetzen des Kontaktglases 1.3 wird die Flüssigkeit 14 zwischen Basiskörper 2.3 und Ansetzkörper 3.3 zusammengepresst und gleichmässig zwischen den beiden Kontaktflächen 6.3, 6.4 verteilt.

Wird zu viel Flüssigkeit 14 aufgetragen, kann die überschüssige Flüssigkeit 14 in den Zwischenraum 12 ausweichen. In den Zwischenraum 12 kann jedoch nicht nur überschüssige Flüssigkeit 14, sondern auch die Luft, die sich zwischen den beiden Kontaktflächen 6.3, 6.4 befindet, entweichen.

Damit befindet sich zwischen dem Basiskörper 2.3 und dem Ansetzkörper 3.3 keine Luft mehr. Wird nun die gelartige Flüssigkeit 14 derart gewählt, dass ihr Brechungsindex in etwa dem Brechungsindex von Basiskörper 2.3, bzw. Ansetzkörper 3.3 entspricht, können, wie bereits erwähnt, die Reflexionen optischer Strahlen an den Grenzübergängen zwischen den verschiedenen Materialen vermindet werden. Mit anderen Worten: Die Kontaktflächen müssen keine optisch hohe Oberflächengüte aufweisen. Insbesondere kann eine teure und hinsichtlich Beschädigungen anfällige Antireflexbeschichtung eliminiert werden. Selbstverständlich werden auch die für Basiskörper 2.3 und Ansetzkörper 3.3 verwendeten Materialen derart gewählt, dass einerseits ihr Brechungsindex gleich oder zumindest fast gleich ist und dieser Brechungsindex ungefähr gleich dem Brechungsindex des Auges ist.

Bei einem Brechungsindex des Auges von etwa 1.33 und einem Kontaktglas mit Brechungsindex 1.5 wird eine Restreflexion von etwa 4 Promille erreicht, wobei Restreflexionen bis etwa 5 Promille kein Problem darstellen. Die gelartige Flüssigkeit 14 wird entsprechend mit einem Brechungsindex im Bereich von etwa 1.3 bis 1.7 gewählt.

Es steht eine grosse Auswahl verwendbarer Flüssigkeiten für dieses Indexmatching zur Verfügung. Eine in der Augenbehandlung bekannte Flüssigkeit ist beispielsweise eine zweiprozentige Lösung von Methylhydroxypropylcellulosum mit Natrii chloridum und Benzalkonii chloridum als Konservierungsmittel, bekannt unter dem Namen Methocel 2%.

Anstatt eine Flüssigkeit 14 für das Indexmatching zu verwenden, können auch andere Stoffe, beispielsweise sogenannte "Solid Gels", verwendet werden. Als Beispiel sei eine transparente, in ihrer Dicke elastisch verformbare Silikonfolie erwähnt, welche vor dem Zusammensetzen des Kontaktglases zwischen die beiden Kontaktflächen eingebracht wird. Um auch hier zu vermeiden, dass Luft zwischen den Kontaktflächen eingeschlossen wird, kann die Folie im Zentrum beispielsweise überhöht oder die Wölbungen der Kontaktflächen entsprechend ausgebildet werden.

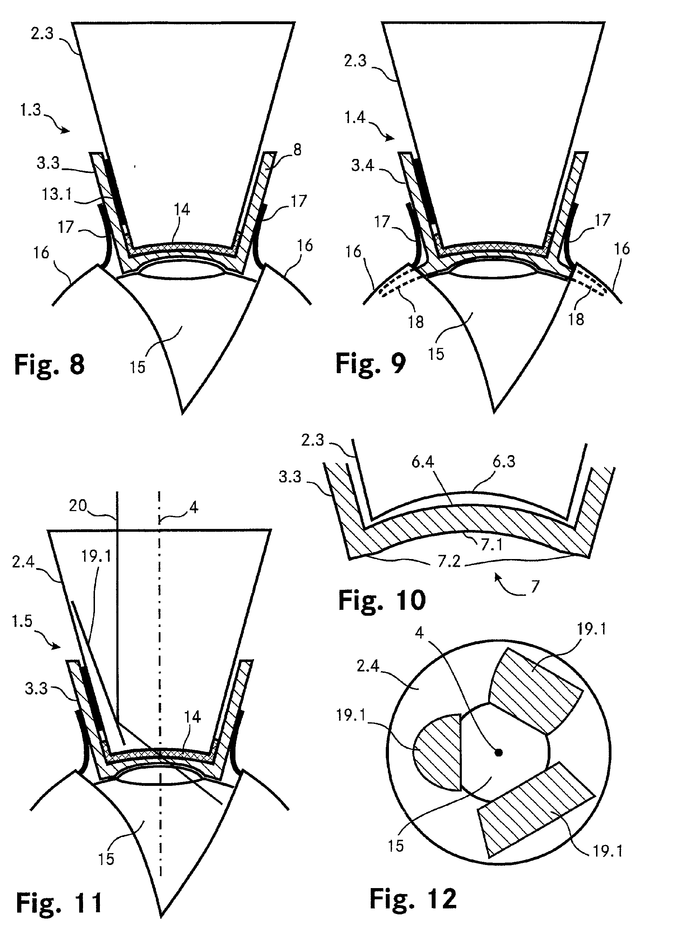

Figur 8 zeigt das Kontaktglas aus Figur 6, wie es auf einem Auge 15 aufgesetzt ist. Dargestellt sind weiter die beiden Augenlider 16 mit den Wimpern 17.

Durch die hochgezogene Seitenwand 8 des Ansetzkörpers 3.3 wird verhindert, dass das Auge 15, die Augenlider 16 oder die Wimpern 17 mit dem Basiskörper 2.3 in Kontakt kommen. D. h. der Basiskörper 2.3 muss nicht nach jedem Gebrauch sterilisiert werden, eine Desinfektion reicht aus. Weiter kann der Ansetzkörper 3.3 derart ausgebildet sein, dass er sterilisiert werden kann. Weniger aufwendig und, da der Ansetzkörper 3.3 auf einfachste Art und Weise, beispielsweise mittels Spritzguss, aus preiswerten Materialen hergestellt werden kann, entsprechend kostengünstig ist es hingegen, den Ansetzkörper 3.3 als Wegwerfartikel zu konzipieren, der nach einmaligem Gebrauch oder nach wenigen Verwendungen weggeworfen wird. Zum Desinfizieren bzw. Sterilisieren stehen ebenfalls eine Vielzahl von bekannten Methoden bzw. Mitteln zur Verfügung.

In Figur 9 ist wiederum das Auge 15 mit Augenlidern 16 und Wimpern 17 mit einem aufgesetzten Kontaktglas 1.4 dargestellt. Dieses weist denselben Basiskörper 2.3 wie das Kontaktglas 1.3 aus Fig. 8, jedoch einen anderen Ansetzkörper 3.4 auf.

Der Ansetzkörper 3.4 ist mit einem sogenannten Skleraring 18 ausgestattet. Dieser wird, wie dargestellt, beim Aufsetzen des Ansetzkörpers 3.4 auf das Auge 15 unter die Augenlider 16 geschoben und wird von diesen in seiner Position festgehalten. Der Skleraring kann entweder gänzlich um den Ansetzkörper herum verlaufen, oder er ist nur in bestimmten Bereichen, beispielsweise im Bereich der Augenlider 16 vorhanden.

in Figur 10 ist ein vergrösserter Ausschnitt des Kontaktglases 1.3 aus Fig. 8 dargestellt, wobei zur besseren Übersicht die gelartige Flüssigkeit 14 weggelassen wurde. Es zeigt die unterschiedlichen Wölbungen der verschiedenen Grenzflächen des Kontaktglases 1.3. Die Kontaktfläche 6.3 des Basiskörpers 2.3 weist beispielsweise einen kleineren Krümmungsradius auf als die Kontaktfläche 6.4. Weiter ist zu erkennen, dass die Berührungsfläche 7 des Ansetzkörpers 3.3 zur optimalen Auflage auf einem Auge zwei unterschiedlich gekrümmte Bereiche 7.1 und 7.2 aufweist. Der zentrale Bereich 7.1 mit dem kleineren Krümmungsradius, welcher ungefähr dem Krümmungsradius der Kontaktfläche 6.4 entspricht, ist zur Positionierung über dem Bereich der Augenpupille gedacht, welcher in der Regel etwas überhöht ist. Der äussere Bereich 7.2 mit grösserem Krümmungsradius kommt entsprechend auf die weniger stark gekrümmten Bereiche rund um die Pupille herum zu liegen.

Es ist jedoch zu betonen, dass auch viele andere Anordnungen mit anderen Krümmungsradien bzw. anders gewählten Krümmungszentren möglich sind. So kann beispielsweise der Krümmungsradius der Kontaktfläche 6.3 grösser sein als jener der Kontaktfläche 6.4. Weiter könnte auch eine oder beide Kontaktflächen 6.3, 6.4 eben ausgebildet sein oder eine Krümmung in entgegengesetzer Richtung aufweisen.

Die Figuren 11 und 12 zeigen ein auf das Auge 15 aufgesetztes Kontaktglas 1.5, mit dem Ansetzkörper aus Fig. 8. Der hier dargestellte Basiskörper 2.4 weist jedoch eine Mehrzahl von seitlich im Basiskörper 2.4 positionierten Spiegeln 19.1, 19.2, 19.3 auf, wobei im Schnittbild in Figur 11 nur der Spiegel 19.1 sichtbar ist. Die Spiegel 19.1, 19.2, 19.3 sind jeweils zur Untersuchung von verschiedenen seitlichen Bereichen des Inneren des Auges 15 ausgebildet und sind entsprechend unterschiedlich geformt bzw. weisen unterschiedliche Neigungswinkel gegenüber der Rotationsachse 4 auf. Die Spiegel 19.1, 19.2, 19.3 werden beispielsweise hergestellt, indem der Basiskörper 2.4 im gewünschten Bereich mit dem entsprechenden Neigungswinkel plangeschliffen wird, wobei mit einer nicht dargestellten Hülle des Basiskörpers 2.4 der Einfall von Fremdlicht verhindert wird.

In Figur 11 ist weiter ein Lichtstrahl 20 dargestellt, der parallel zur Rotationsachse 4 in das Kontaktglas 1.5 eintritt und am Spiegel 19.1 abgelenkt wird, sodass er durch die gelartige Flüssigkeit 14 und den Ansetzkörper 3.3 hindurch in einen seitlichen Bereich des Auges 15 gelenkt wird. Ein solcher in das Auge 15 dringender Lichtstrahl 20 wird beispielsweise zur Beleuchtung des inneren des Auges 15 eingesetzt. Verläuft der Lichtstrahl 20 jedoch in der dargestellten Richtung aus dem Auge heraus durch das Kontaktglas, kann er vom Benutzer beobachtet werden.

Nicht berücksichtigt im Verlauf des Lichtstrahls 20 ist die Brechung an den verschiedenen Grenzschichten des Auges 15 bzw. des Kontaktglases 1.5 resp. dazwischen. Je nach gewählter Geometrie der Bestandteile des Kontaktglases 1.5 können nämlich auch verschiedene optische Effekte wie eine bestimmte Vergrösserung oder auch unterschiedliche Sehfelder innerhalb des Auges 15 erreicht werden.

Um nun den mit dem Spiegel 19.1 beobachtbaren seitlichen Innenbereich des Auges 15 vollständig zu untersuchen, muss der Basiskörper 2.4 lediglich um seine Rotationsachse 4 gedreht werden. Der Ansetzkörper 3.3 bleibt hierbei ohne selber bewegt zu werden auf dem Auge 15 aufgesetzt und kann deshalb auch keine Verletzung am Auge 15 verursachen. Die Flüssigkeit 14 erleichtert diese Drehung des Basiskörpers 2.4 im Ansetzkörper 3.3.

Zusammenfassend ist festzustellen, dass es die Erfindung erlaubt, mit einigen wenigen Basiskörpern und einigen wenigen Arten von Ansetzkörpern eine Vielzahl von Vorrichtungen zur Untersuchung bzw. Behandlung von Augen zusammenzusetzen, anstatt für jede Untersuchung/Behandlung eine eigens dafür vorgesehene Vorrichtung zu verwenden. Zudem kann der Basiskörper, da er bei der Untersuchung bzw. Behandlung nicht mit dem Auge in Kontakt kommt, aus einem Material hergestellt werden, das nicht sterilisierbar sein muss und entsprechend preiswerter sowie einfacher in der Verarbeitung ist.

## Patentansprüche

1. Vorrichtung zur Untersuchung und/oder Behandlung eines Auges, wobei sie auf das Auge aufzusetzen ist, **dadurch gekennzeichnet, dass** sie einen Basiskörper und einen mit dem Basiskörper zusammensetzbaren Ansetzkörper aufweist, wobei die Vorrichtung zur Untersuchung und/oder Behandlung des Auges mit dem Ansetzkörper auf das Auge aufzusetzen ist.

2. Vorrichtung nach Anspruch 1, **dadurch gekennzeichnet, dass** der Basiskörper eine erste Kontaktfläche und der Ansetzkörper eine zweite Kontaktfläche aufweist, wobei die zweite Kontaktfläche vorzugsweise im Wesentlichen formgleich zur ersten Kontaktfläche ausgebildet ist, der Ansetzkörper insbesondere derart mit dem Basiskörper zusammensetzbar ist, dass die Kontaktflächen im Wesentlichen parallel zueinander liegen und dass der Basiskörper und der Ansetzkörper in bevorzugter Weise derart ausgebildet sind, dass sie, insbesondere um eine optische Achse, gegeneinander verdrehbar sind.

3. Vorrichtung nach Anspruch 1 oder 2, **dadurch gekennzeichnet, dass** der Basiskörper bzw. der Ansetzkörper ausserhalb der ersten bzw. der zweiten Kontakfläche Distanzierungselemente, insbesondere eine Mehrzahl von Erhebungen, zur Wahrung eines Mindestabstandes zwischen dem Basiskörper und dem Ansetzkörper, und die zusammengesetzte Vorrichtung zwischen der ersten und der zweiten Kontaktfläche vorzugsweise ein optisch transparentes, verformbares Zwischenmaterial aufweist.

4. Vorrichtung nach einem der Ansprüche 1 bis 3, **dadurch gekennzeichnet, dass** die zusammengesetzte Vorrichtung derart ausgebildet ist, dass, wenn sie auf das Auge eines Patienten aufgesetzt ist, ausschliesslich der Ansetzkörper mit dem Patienten in Kontakt ist und der Ansetzkörper vorzugsweise mittels einer lösbaren, formund/oder kraftschlüssigen Verbindung am Basiskörper gehalten ist.

5. Basiskörper für eine Vorrichtung nach Anspruch 1, **dadurch gekennzeichnet, dass** er für eine ophtalmologische, insbesondere optische Untersuchung und/oder Behandlung des Auges ausgebildet ist.

6. Basiskörper nach Anspruch 5, **gekennzeichnet durch** einen Spiegel zur Untersuchung und/oder Behandlung eines seitlichen Innenbereiches oder eines Kammerwinkels des Auges.

7. Ansetzkörper, insbesondere für eine Vorrichtung nach Anspruch 1, **dadurch gekennzeichnet, dass** er zum Aufsetzen auf ein Auge eine im Wesentlichen an einen Oberflächenbereich des Auges angepasste Berührungsfläche aufweist und der Ansetzkörper insbesondere desinfizierbar oder sterilisierbar ausgebildet ist.

8. Ansetzkörper nach Anspruch 7, **dadurch gekennzeichnet, dass** die Berührungsfläche derart ausgebildet ist, dass sie unter zumindest ein Augenlid des Patienten schiebbare Randbereiche aufweist.

9. Set von Ansetzkörpern nach Anspruch 7, **dadurch gekennzeichnet, dass** die Ansetzkörper gebrauchsfertig und einzeln, insbesondere steril, verpackt in einem Behälter untergebracht und vorzugsweise derart im Behälter gehalten sind, dass ein Zusammensetzen mit einem Basiskörper ohne haltende Berührung eines Ansetzkörpers mit wahlweisem Einbringen eines optisch transparenten, verformbaren Zwischenmaterials zwischen Basiskörper und Ansetzkörper möglich ist.

10. Verfahren zum Zusammensetzen einer Vorrichtung, insbesondere einer Vorrichtung nach Anspruch 1, umfassend einen Basiskörper, insbesondere einen Basiskörper nach Anspruch 5 und einen Ansetzkörper, insbesondere einen Ansetzkörper nach Anspruch 7 oder einen Ansetzkörper aus einem Set nach Anspruch 9, **dadurch gekennzeichnet, dass** der Ansetzkörper mit dem Basiskörper zusammengesetzt wird, wobei vor dem Zusammensetzen ein optisch transparentes, verformbares Zwischenmaterial zwischen den Basiskörper und den Ansetzkörper eingebracht wird.
